# EUROPÄISCHE PATENTSCHRIFT

(11) **EP 1 004 869 B1**
(45) Veröffentlichungstag und Bekanntmachung des Hinweises auf die Patenterteilung: **14.04.2010**
(21) Anmeldenummer: 99250252.6
(22) Anmeldetag: 28.07.1999
(51) Int. Cl.: G01N 1/10, G01N 1/28, G01N 35/00

(54) **Verfahren und Vorrichtung zum Behandeln von Proben**
Method and apparatus for handling samples
Procédé et dispositif pour manipuler des échantillions

(30) Priorität: 06.11.1998 DE 19852528
(43) Veröffentlichungstag der Anmeldung: 31.05.2000
(73) Patentinhaber: SMS Siemag AG, 40237 Düsseldorf (DE)
(72) Erfinder: Schöck, Hans-Wilhelm, Ing., 47239 Duisburg (DE); Rose, Lutz, 47259 Duisburg (DE)
(74) Vertreter: Meissner, Peter E.

(56) Entgegenhaltungen:
- DE-A1- 19 654 522
- US-A- 4 058 017
- Patent Abstracts of Japan, Band 94, Juli 1994; & JP 06 201541 A (OSAKA OXYGEN IND LTD), 19. Juli 1994.

## Beschreibung

Die Erfindung betrifft ein Verfahren zum Behandeln von Proben, die sich in einer Meßsonde befinden, welche, nachdem sie in eine sich in einem metallurgischen Gefäß, insbesondere einen Konverter, befindliche Stahlschmelze getaucht wurden, mittels eines Manipulators von einem Kontaktrohr einer Meßlanze abgezogen werden, sowie eine Einrichtung zur Durchführung des Verfahrens.

Aus JP-OS 6-201541 ist ein Verfahren zum Entfernen von Proben aus einer Meßsonde bekannt, bei dem eine Trenneinrichtung zum Einsatz kommt, die einen Teilbereich aus der Meßsonde herausschneidet, in dem sich die Probe befindet. Im nachfolgenden Schritt wird die Probe aus der Restmeßsonde entfernt und über eine Rohrpostanlage einem Analysengerät zugeführt.

Aus der DE 196 54 522 A1 ist ferner eine Einrichtung bekannt, bei der die Sonde nach ihrem Ablegen von einem Manipulator in eine Trennstation von dem Greifer eines Handhabungsgerätes im Bereich der Probe gegriffen wird. Anschließend wird die Sonde durch eine Trenneinrichtung zu beiden Seien des Greifers abgetrennt und die Probe aus dem Restsondenteil herausgedrückt.

Bei der Fülle der hier bis zur endgültigen Analyse erforderlichen Schritte vergeht wertvolle Zeit.

Die Erfindung hat sich daher das Ziel gesetzt, eine Einrichtung zur Analyse von Proben, insbesondere von Stahlproben für Konverter, zu schaffen, bei der ein Minimum von Tätigkeiten erforderlich ist, um zur fertigen Analyse zu gelangen.

Die Erfindung erreicht dieses Ziel durch die kennzeichnenden Merkmale des Verfahrensanspruches 1 und des Vorrichtungsanspruches 5. Die übrigen Ansprüche sind vorteilhafte Weiterbildungen der Erfindung.

Erfindungsgemäß wird die Probe einem Analysengerät zugeführt, beispielsweise einem Emissions-Spektrometer oder einem Laser-Analysengerät. Vom Analysengerät wird der Aktivierungsstrahl, beim Laser-Analysengerät also der Laserstrahl, auf die freie Oberfläche des Probenteils gerichtet, welches sich noch in der Meßsonde befindet. Die Meßsonde ist zur Vorbereitung der Probennahme in dem Bereich, in dem sich die Probe befindet, einschließlich dieser durchgetrennt worden und der Meßsondenstumpf wurde anschließend mit dem von ihm gehaltenen Probenteil dem Analysengerät zugeführt. Bei Einsatz eines Laser-Analysengerätes wird hier ein Plasma erzeugt, welches zur Durchführung von Analysen genutzt wird.

Die Meßsonde, die insbesondere in eine in einem Konverter befindliche Stahlschmelze getaucht wurde, wird mittels eines Manipulators von einem Kontaktrohr einer Meßlanze abgezogen und von demselben Manipulator unmittelbar zu einer Trennstation geführt. Diese Meßsonde wird hier ohne weitere Vorbereitung der Schnittfläche der Metallprobe in der Weise unter einem Analysengerät positioniert, daß die Metallprobe durch einen Aktivierungsstrahl in der Weise aktiviert wird, daß an der Oberfläche der Metallprobe ein Aggregatzustand erzeugt wird und die Auswertung der reflektierten Strahlung zu einer Komplettanalyse der Stahlschmelze geführt.

Die schnell Verfügbarkeit der Schmelzenanalyse, insbesondere die Ermittlung der Phosphorgehalte, ist maßgebend für die Entscheidung, ob direkt abgestochen werden kann oder ob die Schmelze weiter behandelt werden muß.

In einer vorteilhaften Weise wird der Meßsondenstumpf in der Weise umpositioniert, daß die an seiner Außenseite befindliche Schlacke ebenfalls dem Analysengerät zugeführt wird. Bei Einsatz eines Laser-Analysengerätes wird hierbei der Laserstrahl auf verschiedene Stellen der Schlackeschicht gerichtet, wobei zur Analyse ein nutzbares Plasma erzeugt wird und mit Sicherheit eine ausreichend starke Schicht an Schlacke erfaßt wird. Dabei wird in einer Ausgestaltung die Außenwandung der Meßlanze in der Weise präpariert, daß in besonders sicherer Weise eine ausreichende Menge von Schlacke von außen anhaftet.

Als Trenneinrichtung zum Durchtrennen der Meßsonde einschließlich der Probe wird als mechanisches Verfahren eine Trennscheibe und als thermisches Verfahren der Einsatz eines Plasmastrahls vorgeschlagen.

Da Ungenauigkeiten insbesondere bei der Übernahme durch den Manipulator entstehen können, wird vorgeschlagen, vor dem Durchtrennen der Meßsonde die exakte Lage der Probe in der Meßsonde zu orten und die Daten zur Positionierung der Meßsonde in der Trennvorrichtung zu verwenden.

Zur Durchführung des Verfahrens der Vor-Ort-Analyse von Metall und Schlackeschmelzen wird erfindungsgemäß eine Einrichtung vorgeschlagen, bei der eine Haltevorrichtung vorgesehen ist, in der die Meßsonde vom Manipulator vertikal einführbar ist, so daß die Trennvorrichtung in der Nähe oberhalb der Haltevorrichtung einsetzbar ist und die Meßvorrichtung von der Haltevorrichtung in einer Position gehalten wird, daß die in der Meßsonde befindliche Probe von der Trennvorrichtung etwa halbiert wird. Nach der Durchtrennung der Meßsonde wird die Mündung des Meßsondenstumpfes durch den Manipulator zum Analysengerät geführt, wobei das Analysengerät beispielsweise eine Lasereinrichtung besitzt, mit der der Laserstrahl auf die freie Oberfläche der im Meßsondenstumpf befindlichen Probe richtbar ist. Nach der Analyse der Metallprobe wird durch den Manipulator der Meßsondenstumpf, und hier insbesondere die präparierte Stelle der Meßsonde dem Analysator zugeführt.

Die Außenwandung des Bereichs der Schlackenprobennahme wird in einer vorteilhaften Ausgestaltung mechanisch bearbeitet, wobei diese Bereiche im Vergleich zur Rest-Meßsonde eine besondere Rauhigkeit aufweist. In einer weiteren Ausgestaltung ist die Außenwandung im Bereich der Schlackenprobennahme chemisch behandelt. Zum Einsatz kommt hier eine insbesondere thermisch resistente Masse zum Einsatz, die eine möglichst große Oberfläche aufweist. Vorgeschlagen wird hier eine Schicht aus einer keramischen Masse.

Ein Beispiel der Erfindung ist in der beigefügten Zeichnung dargelegt. Dabei zeigt die
- Figur 1: den Arbeitsschritt: Trennen,
- Figur 2: eine Trennvorrichtung,
- Figur 3: den Arbeitsschritt: Analyse der Probe,
- Figur 4: den Arbeitsschritt: Analyse Schlacke,
- Figur 5: den Schlackebereich einer Meßsonde.

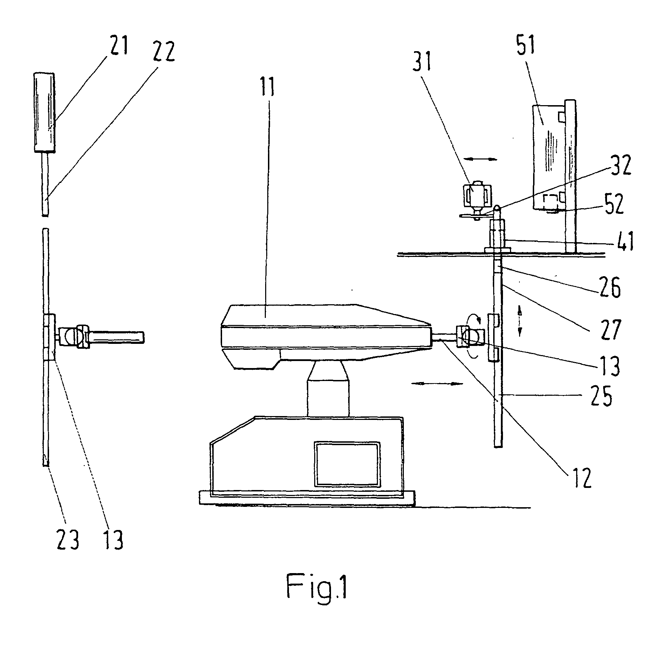

Die Figur 1 zeigt in der Übersicht die Einrichtung zum Behandeln von Proben mit einer Meßlanze 21, von deren Kontaktrohr 22 eine Meßsonde 23 abgezogen wurde. Die Meßsonde 23 wird dabei von einem an einen Schwenkarm 12 eines Manipulators 11 angeordneten Greifer 13 gegriffen und zu einer Halteeinrichtung 41 geführt.

Im Bereich der Halteeinrichtung 41 ist eine Trennvorrichtung 31 vorgesehen, mit deren Trennwerkzeug 32 die Probe 29 in der Weise durchtrennt wird, daß eine Hälfte der Probe 29 im Meßsondenstumpf 25 verbleibt. Darüber hinaus ist am Meßsondenstumpf 25 an der Außenwandung 27 ein Bereich 26 zu erkennen, an dem bevorzugt Schlacke anhaftet.

In Greifnähe des Schwenkarmes 12 ist ein Analysengerät 51 angeordnet, das eine Aktivierungseinrichtung 52 zur Durchführung der Analyse besitzt, beispielsweise eine Laser-Einrichtung.

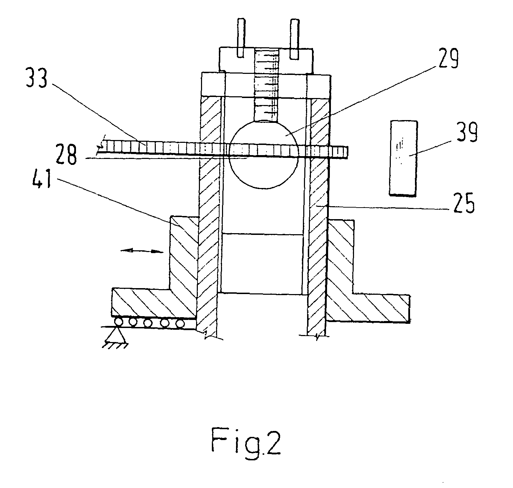

In der Figur 2 ist die Halteeinrichtung 41 dargestellt, die den Meßsondenstumpf 25 festhält, und zwar in der Weise, daß das Trennwerkzeug, hier eine Trennscheibe 33, die Probe 29 halbiert.

Zur exakten Lageerkennung des Meßsondenstumpfes 25 in Relation zur Trennscheibe 33 ist ein Detektor 39 vorgesehen.

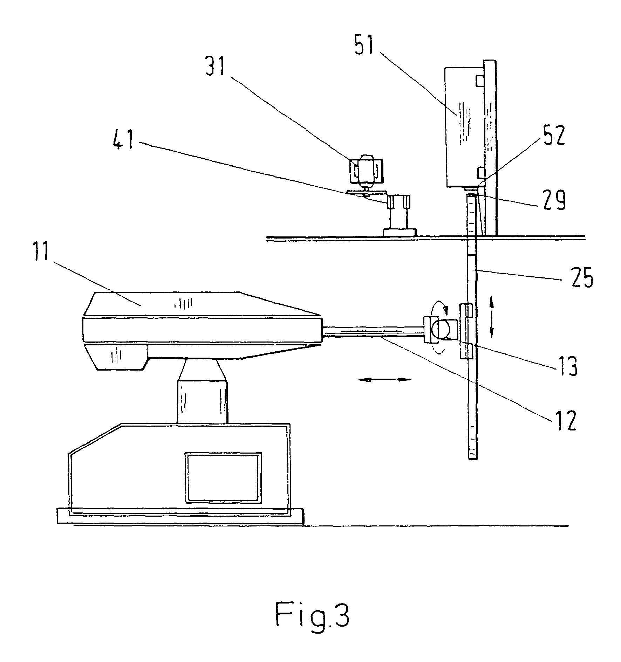

In der Figur 3 ist mittels des Schwenkarmes 12 des Manipulators 11 der Meßsondenstumpf 25 zum Analysengerät 51 geführt worden, wobei er so positioniert ist, daß die Probe 29 mit ihrer freien Oberfläche 28 (siehe hierzu Figur 2) mit der Aktivierungseinrichtung 52 korrespondiert.

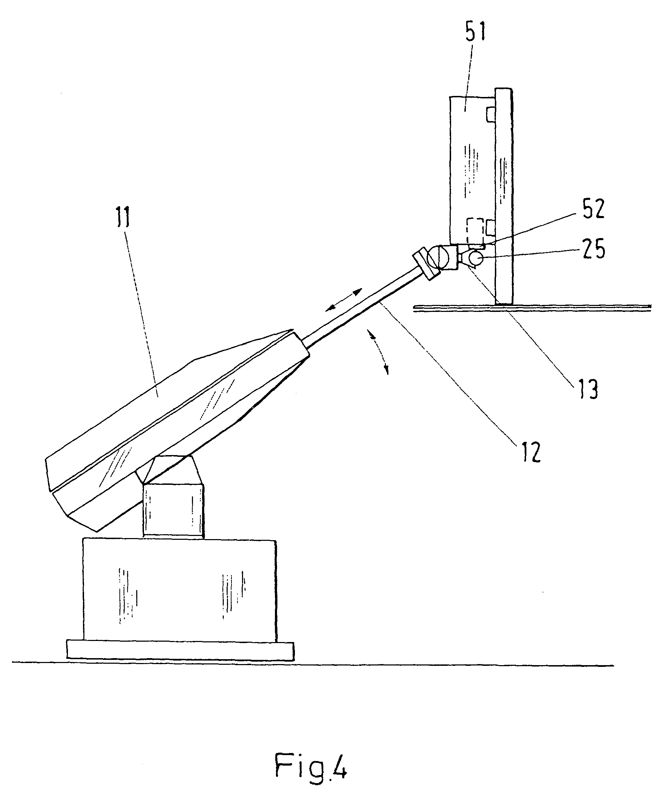

In der Figur 4 ist der Manipulator 11 so verschwenkt, daß der am Schwenkarm 12 befestigte Greifer den Meßsondenstumpf 25 in eine horizontale Lage gebracht hat und in der Weise zum Analysengerät 51 geführt hat, daß die Außenwandung 27 des Meßsondenstumpfes 25 mit der Aktivierungseinrichtung 52 korrespondiert.

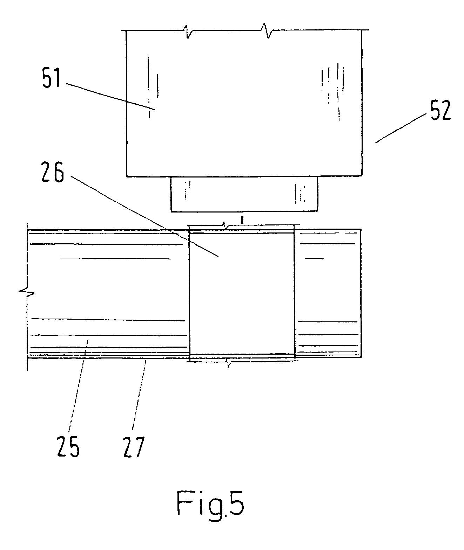

In der Figur 5 ist im Detail gezeigt, wie der Meßsondenstumpf 25, der an der Außenwandung 27 einen Bereich 26 aufweist, an dem sich in besonderem Maße Schlacke angesammelt hat, welche der Aktivierungseinrichtung 52 am Analysengerät 51 zugeführt wird.

### Positionsliste

### Manipulieren

- 11: Manipulator
- 12: Schwenkarm
- 13: Greifer

### Sonde

- 21: Meßlanze
- 22: Kontaktrohr
- 23: Meßsonde
- 24: Mündung vom Stumpf
- 25: Meßsondenstumpf
- 26: Bereich der Meßsonde
- 27: Außenwandung
- 28: Freie Oberfläche der Probe
- 29: Probe

### Trennen

- 31: Trennvorrichtung
- 32: Trennwerkzeug
- 33: Trennscheibe
- 34: Lasereinrichtung zum Trennen
- 39: Detektor

### Halten

- 41: Halteeinrichtung

### Analysieren

- 51: Analysengerät
- 52: Aktivierungseinrichtung zur Analyse

## Patentansprüche

1. Verfahren zum Behandeln von Proben, die sich in einer Meßsonde befinden, welche, nachdem sie in eine sich in einem metallurgischen Gefäß, insbesondere einen Konverter, befindliche Stahlschmelze getaucht wurden, mittels eines Manipulators von einem Kontaktrohr einer Meßlanze abgezogen werden, **gekennzeichnet durch** folgende Schritte:
a) die Meßsonde wird in dem Bereich, in dem sich die Probe befindet, einschließlich dieser durchtrennt,
b) anschließend wird der eine Meßsondenstumpf mit dem von ihm gehaltenen Probenteil einem Analysengerät zugeführt,
c) von demAnalysengerat wird die Trennstelle der Probe zur Durchführung der Analyse benutzt.

2. Verfahren nach Anspruch 1,
**dadurch gekennzeichnet,**
**daß** nach der Analyse des Probenteils der Meßsondenstumpf in der Weise umpositioniert wird, daß an seiner Außenseite befindliche Schlacke dem Analysengerät zugeführt wird, und
**daß** von verschiedenen Stellen der Schlackeschicht Analysen genommen werden.

3. Verfahren nach Anspruch 1,
**dadurch gekennzeichnet,**
**daß** das Durchtrennen der Meßsonde thermisch, insbesondere durch ein Plasmastrahl erfolgt.

4. Verfahren nach Anspruch 1,
**dadurch gekennzeichnet,**
**daß** die exakte Lage der Probe in der Meßsonde geortet wird und die Daten zur Positionierung der Meßsonde in der Trennvorrichtung verwendet werden.

5. Einrichtung zum Behandeln von Proben, die sich in Meßsonden befinden, welche in eine sich in einem metallurgischen Gefäß, insbesondere Konverter, befindliche Stahlschmelze getaucht werden, mit einem Manipulator, der die Meßsonde in vorgebbare Positionen führt, einer Trennvorrichtung und einem Analysengerät zur Durchführung des Verfahrens nach Anspruch 1, **dadurch gekennzeichnet,**
**daß** eine Halteeinrichtung (41) für die Meßsonde (23)vorgesehen ist, in die sie vom Manipulator (11) in der Weise vertikal einführbar ist, daß die Trennvorrichtung (31) in der Nähe oberhalb der Halteeinrichtung (41) einsetzbar ist,
**daß** die Meßsonde (23) von der Halteeinrichtung (41) in einer Position gehalten wird, daß die in der Meßsonde (23) befindliche Probe (29) von der Trennvorrichtung (31) in etwa halbiert wird,
**daß** nach Durchtrennung der Meßsonde (23) die Mündung (24) des Meßsondenstumpfes (25) durch den Manipulator (11) zum Analysengerät (51) führbar ist, und
**daß** das Analysengerät (51) eine Aktivierungseinrichtung (52) besitzt, mit der von der freien Oberfläche (28) der Probe (29) die Analyse durchführbar ist.

6. Einrichtung nach Anspruch 5,
**dadurch gekennzeichnet,**
**daß** das Analysengerät ein Emission-Spektrometer ist.

7. Einrichtung nach Anspruch 5,
**dadurch gekennzeichnet,**
**daß** das Analysengerät eine Lasereinnchtung besitzt, mit der ein Laserstrahl auf die freie Oberfläche (28) der Probe (29) richtbar ist.

8. Einrichtung nach Anspruch 5,
**dadurch gekennzeichnet,**
**daß** der Manipulator (11) in der Weise bewegbar ist, daß der Meßsondenstumpf (25) ergänzend zur vertikalen auch in eine horizontale Lage führbar und dabei so positionierbar ist, daß von der Aktivierungseinrichtung (52) ein Aktivierungsstrahl auf einen Bereich (26) der Außenwandung (27) an der Meßsonde (23) richtbar ist, an dem Schlacke anhaftet.

9. Einrichtung nach Anspruch 5,
**dadurch gekennzeichnet,**
**daß** das Trennwerkzeug (32) der Trennvorrichtung (31) eine Trennscheibe ist.

10. Einrichtung nach Anspruch 5,
**dadurch gekennzeichnet,**
**daß** das Trennwerkzeug (32) der Trennvorrichtung (31) eine Lasereinrichtung ist.

11. Einrichtung nach Anspruch 5,
**dadurch gekennzeichnet,**
**daß** im Bereich der Trennvorrichtung (31) ein Detektor (39) vorgesehen ist, mit dem die exakte Lage der metallischen Probe (29) in der Meßsonde (23) ermittelbar ist.

## Claims

1. A method of processing samples located in a measuring probe which, after it has been dipped into a steel melt located in a metallurgical vessel, in particular a converter, can be removed by means of a manipulator from a contact tube of a measuring lance, **characterised by** the following steps:
a. the measuring probe, together with the sample, is cut in two in the region in which the sample is located,
b. then one measuring-probe stub together with the sample portion held by the latter is supplied to an analyzer,
c. the severance site of the sample is used by the analyzer to carry out the analysis.

2. A method according to claim 1, **characterised in that** after analysis of the sample portion, the measuring-probe stub is repositioned in such a manner that slag located on its exterior is supplied to the analyzer, and **in that** analyses of different sites of the slag layer are made.

3. A method according to claim 1, **characterised in that** the cutting in two of the measuring probe takes place thermally, in particular by a plasma beam.

4. A method according to claim 1, **characterised in that** the exact position of the sample in the measuring probe is located and the data used to position the measuring probe in the severing device.

5. A device for processing samples located in measuring probes which are dipped into a steel melt located in a metallurgical vessel, in particular a converter, having a manipulator which guides the measuring probe into predeterminable positions, a severing device and an analyzer for carrying out the method according to claim 1,
**characterised in that** a holding device (41) for the measuring probe (23) is provided, into which the latter can be inserted vertically by the manipulator (11) in such a manner that the severing device (31) can be deployed in the vicinity above the holding device (41),
**in that** the measuring probe (23) is held by the holding device (41) in a position such that the sample (29) located in the measuring probe (23) is substantially halved by the severing device (31),
**in that** after cutting in two of the measuring probe (23), the aperture (24) of the measuring-probe stub (25) can be guided to the analyzer (51) by the manipulator (11), and
**in that** the analyzer (51) has an activating device (52) by means of which the analysis of the free surface (28) of the sample (29) can be carried out.

6. A device according to claim 5, **characterised in that** the analyzer is an emission spectrometer.

7. A device according to claim 5, **characterised in that** the analyzer has a laser device by means of which a laser beam can be directed onto the free surface (28) of the sample (29).

8. A device according to claim 5, **characterised in that** the manipulator (11) is movable in such a manner that the measuring-probe stub (25) can in addition to the vertical position also be guided into a horizontal position and in the process can be positioned in such a manner that an activating beam can be directed by the activating device (52) onto a region (26) of the outer wall (27) of the measuring probe (23) to which slag adheres.

9. A device according to claim 5, **characterised in that** the severing tool (32) of the severing device (31) is a cutting wheel.

10. A device according to claim 5, **characterised in that** the severing tool (32) of the severing device (31) is a laser device.

11. A device according to claim 5, **characterised in that** a detector (39) is provided in the region of the severing device (31), by means of which detector the exact position of the metallic sample (29) in the measuring probe (23) can be determined.

## Revendications

1. Procédé de traitement d'échantillons qui se trouvent dans une sonde de mesure et qui, après avoir été plongés dans un bain d'acier liquide se trouvant dans une cuve métallurgique, en particulier un convertisseur, en sont retirés au moyen d'un manipulateur d'un tube de contact d'une lance de mesure, **caractérisé par** les étapes suivantes :
a) la sonde de mesure est séparée en deux dans la zone où se trouve l'échantillon, de même que celui-ci,
b) l'un des tronçons de la sonde de mesure avec la partie d'échantillon retenue par ce dernier est ensuite acheminé à un appareil d'analyse,
c) le point de séparation de l'échantillon est utilisé pour réaliser l'analyse par l'appareil d'analyse.

2. Procédé selon la revendication 1,
**caractérisé en ce que**, après l'analyse du tronçon d'échantillon, le tronçon de sonde de mesure est repositionné de manière à acheminer des scories se trouvant sur sa face externe à l'appareil d'analyse, et
des analyses sont faites de divers points de la couche de scories.

3. Procédé selon la revendication 1,
**caractérisé en ce que** la séparation de la sonde de mesure s'effectue par voie thermique, en particulier par un rayon plasma.

4. Procédé selon la revendication 1,
**caractérisé en ce que** la position exacte de l'échantillon dans la sonde de mesure est localisée et les données de positionnement de la sonde de mesure sont utilisées dans le dispositif de séparation.

5. Dispositif de traitement d'échantillons, qui se trouvent dans des sondes de mesure et qui sont plongés dans un bain d'acier liquide se trouvant dans une cuve métallurgique, en particulier un convertisseur, comprenant un manipulateur, qui guide la sonde de mesure dans des positions prédéterminables, un dispositif de séparation et un appareil d'analyse pour réaliser le procédé selon la revendication 1,
**caractérisé en ce qu'**il est prévu un dispositif de maintien (41) pour la sonde de mesure (23), dans lequel elle peut être introduite verticalement par le manipulateur (11) de sorte que le dispositif de séparation (31) soit utilisable dans le voisinage au-dessus du dispositif de maintien (41),
la sonde de mesure (23) est maintenue par le dispositif de maintien (41) dans une position telle que l'échantillon (29) se trouvant dans la sonde de mesure (23) soit à peu près réduit de moitié par le dispositif de séparation (31),
après la séparation de la sonde de mesure (23), l'embouchure (24) du bout de sonde de mesure (25) peut être guidée par le manipulateur (11) à l'appareil d'analyse (51), et
l'appareil d'analyse (51) possède un dispositif d'activation (52), à l'aide duquel l'analyse est réalisable à partir de la surface libre (28) de l'échantillon (29).

6. Dispositif selon la revendication 5,
**caractérisé en ce que** l'appareil d'analyse est un spectromètre d'émission.

7. Dispositif selon la revendication 5,
**caractérisé en ce que** l'appareil d'analyse possède un dispositif à laser, à l'aide duquel un rayon laser peut être dirigé sur la surface libre (28) de l'échantillon (29).

8. Dispositif selon la revendication 5,
**caractérisé en ce que** le manipulateur (11) peut être déplacé de sorte que le tronçon de sonde de mesure (25) puisse être guidé de façon complémentaire à la verticale, également en position horizontale et y être positionné de manière que le dispositif d'activation (52) puisse diriger un rayon d'activation sur une zone (26) de la paroi externe (27) de la sonde de mesure (23), sur laquelle adhèrent des scories.

9. Dispositif selon la revendication 5,
**caractérisé en ce que** l'outil de séparation (32) du dispositif de séparation (31) est un disque de séparation.

10. Dispositif selon la revendication 5,
**caractérisé en ce que** l'outil de séparation (32) du dispositif de séparation (31) est un dispositif à laser.

11. Dispositif selon la revendication 5,
**caractérisé en ce que** il est prévu dans la zone du dispositif de séparation (31) un détecteur (39), à l'aide duquel peut être déterminée la position exacte de l'échantillon métallique (29) dans la sonde de mesure (23).
